# EUROPEAN PATENT APPLICATION

(11) **EP 4 119 672 A1**
(43) Date of publication of application: **18.01.2023**
(21) Application number: 21185500.2
(22) Date of filing: 14.07.2021
(51) Int. Cl.: C12P 21/00, C12N 15/113

(54) **METHOD OF MODULATING THE EXTENT OF GALACTOSYLATION OF PROTEINS IN MAMMALIAN PRODUCER CELLS**

(71) Applicant: Sartorius Stedim Cellca GmbH, 89081 Ulm (DE)
(72) Inventor: Leroux, Ann-Cathrin, 89231 Neu-Ulm (DE); Zehe, Christoph, 89584 Ehingen (Donau) (DE)
(74) Representative: Wohlfahrt, Jan Günther

(57) **Abstract**

The present invention pertains to the modulation of the extent of glycosylation of recombinantly expressed proteins of interest by modulating the level of at least one miRNA in mammalian producer cells.

## Description

The present invention pertains to the modulation of the extent of glycosylation of recombinantly expressed proteins of interest by modulating the level of at least one miRNA in mammalian producer cells.

Since the approval of recombinant human plasminogen activator in 1987, the first protein expressed in Chinese hamster ovary (CHO) cells, there has been tremendous improvement in biotechnological production concerning cell line development, media optimization and cultivation techniques in order to increase to productivity of mammalian producer cells over 10 g/L. The main focus in cell line development is nowadays not restricted to a achieve a further increase in productivity but also to product quality, such as to obtain a recombinantly produced protein of interest having the desired post-translational modification. The ability of mammalian cells to properly fold complex proteins, to correctly assemble multimeric proteins and protein complexes and in particular their capability to modify target proteins post-translationally are considered the main advantages over the use of prokaryotic expression systems. Typical post-translational modifications of peptides and proteins include hydroxylation, glycosylation, phosphorylation, acetylation, lipidation and the formation of disulfide bonds. The glycosylation of recombinant proteins is of critical importance, in particular if recombinant proteins are destined for administration to human subjects. This becomes especially evident in light of the fact that more than 50% of human proteins are glycosylated (Walsh et al., Post-translational modifications in the context of therapeutic proteins. Nat. Biotechnol., 2006, 24, 1241―1252). The two most frequently occuring types of glycosylation are N-linked and O-linked glycosylation. N-linked glycosylation is characterized by the formation of a covalent bond between the glycan and the amidic group of an asparagine (Asn) residue. In O-linked glycosylated proteins, the glycan is linked to the hydroxyl group of a serine (Ser) or threonine (Thr) residue. The type and extent of glycosylation is known to profoundly affect biological activity, function, clearance from circulation, and crucially immunogenicity.

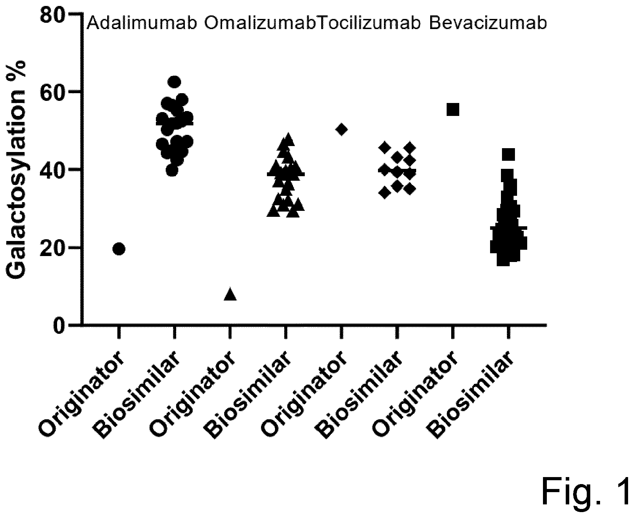

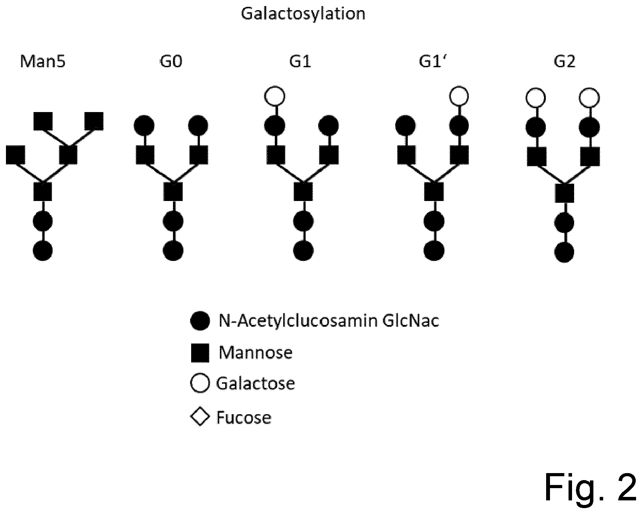

During the development of cell lines for the biotechnological production of biosimilars it is important that the quality parameters correspond to those of the originator. This concerns for example the recombinant production of antibodies (see Fig. 1). Monoclonal antibodies are naturally glycosylated at the L-Asparagine residue in the F_{c} part of the antibody. The glycan structure is among others defined by the terminal galactosylation (G1; G1'; G2) (see Fig. 2). Especially with regard to immunogenicity and cytotoxicity, it is therefore desirable obtain an extent of galactosylation of recombinantly produced biosimilars which matches the glycan profile of the originator as close as possible. In cases in which the originator has a higher extent of galactosylation than the recombinantly produced biosimilar, the extent of galactosylation of the biosimilar can be increased by the addition of galactose to the culture medium. However, in the opposite case, namely when the biosimilar exhibits a higher extent of galactosylation than the originator, there is presently no possibility to reduce the extent of galactosylation.

Accordingly, there is an urgent need for methods of regulating the extent of post-translational glycosylation of recombinantly produced target proteins, in particular methods for the production of glyco-engineered recombinant target proteins in mammalian cells.

The problem underlying the present invention is solved by the subject-matter of the independent claims, in particular by modulating in a mammalian producer cell the level of at least one miRNA capable of modifying the extent of galactosylation of a protein of interest produced by the mammalian producer cell.

The present invention in particular pertains to a method for the production of a protein of interest comprising the steps:
a) providing a mammalian producer cell,
b) modulating in the mammalian producer cell provided in step a) the level of at least one miRNA selected from miRNAs being capable of modifying the extent of galactosylation of a protein of interest produced by the mammalian producer cell,
c) cultivating the mammalian producer cell with the modulated level of the at least one miRNA of step b) under conditions suitable for the production of the protein of interest so as to obtain the protein of interest having a desired extent of galactosylation.

Accordingly, in the method according to the present invention a mammalian producer cell is provided in step a). Subsequently, the level of at least one miRNA selected from miRNAs which are capable of modifying the extent of galactosylation of a protein of interest produced by the mammalian producer cell is modulated, in particular is increased or reduced. The mammalian producer cell having a modulated level of the at least one miRNA, in particular an increased or reduced level of the at least one miRNA, is then cultivated under conditions suitable for the production of a protein of interest so as to obtain the protein of interest having a desired extent of galactosylation. The method therefore advantageously allows for the recombinant production of a protein of interest having a particular extent of galactosylation, preferably a particular extent of galactosylation of the terminal N-linked glycan structure. By a suitable modulation of the level of at least one miRNA selected from miRNAs being capable of modifying the extent of galactosylation of a protein of interest produced by the mammalian producer cell it is possible to modify, that means to increase or reduce, the extent of glycosylation, preferably the extent of galactosylation of the terminal N-linked glycan structure, in a protein of interest in comparison to the extent of glycosylation, preferably the extent of galactosylation of the terminal N-linked glycan structure, of the protein of interest obtained from the mammalian producer cell without the respective modulation in the level of the at least one miRNA. In this way, it is for example possible to adjust the extent of galactosylation, preferably the extent of galactosylation of the terminal N-linked glycan structure, of a recombinantly produced biosimilar to the extent of galactosylation, preferably the extent of galactosylation of the terminal N-linked glycan structure, of a naturally occurring originator. This tremendously reduces the screening efford during cell line development as a significantly reduced number of clones must be analysed to identify a clone producing a biosimilar which has an extent of galactosylation, in particular an extent of galactosylation of the terminal N-linked glycan structure, comparable to that of the originator. Thus, the present invention provides a faster and more economical way of producing a protein of interest having a particular extent of galactosylation than the processes known in the art. Moreover, the method according to the present invention allows the production of a protein of interest, such as a biosimilar, having a relatively low extent of galactosylation which cannot be reached at all in the mammalian producer cell without modulation of the level of at least one miRNA capable of modifying the extent of galactosylation of a protein of interest produced by the mammalian producer cell.

In a preferred embodiment of the present invention, the method for the production of a protein of interest comprises a further step d) of recovering the protein of interest produced in step c).

The recovery of the protein of interest in step d) includes the isolation of the protein from a composition comprising several components, such as from a culture medium.

The step of recovering the protein of interest produced in step c) preferably includes at least one isolation technique selected from centrifugation, in particular differential centrifugation and/or density gradient centrifugation, chromatographic methods, in particular gel filtration chromatography, ion exchange chromatography, affinity chromatography and/or high-performance liquid chromatography (HPLC), electrophoretic methods, filtration methods and/or extraction methods.

Particularly preferred, the step of recovering the protein of interest produced in step c) includes the concentration and purification of the protein of interest.

In a preferred embodiment of the present invention, the step of recovering the protein of interest produced in step c), in particular the concentration and purification of the protein of interest, includes at least two isolation techniques, preferably at least three isolation techniques.

In further preferred embodiment, the modulation in step b) is an increase or reduction in the level of at least one miRNA capable of modifying the extent of galactosylation of a protein of interest produced by the mammalian producer cell.

Preferably, the modulation in step b) is an increase in the level of at least one miRNA capable of modifying the extent of galactosylation of a protein of interest produced by the mammalian producer cell.

In another preferred embodiment of the present invention, the modulation in step b) is a reduction in the level of at least one miRNA capable of modifying the extent of galactosylation of a protein of interest produced by the mammalian producer cell.

In a preferred embodiment of the present invention, the protein of interest obtained in step c) has a reduced extent of galactosylation, in particular extent of terminal N-linked galactosylation, compared to a protein of interest produced in a mammalian producer cell without a modulated level of at least one miRNA selected from the group consisting of miRNAs being capable of modifying the extent of galactosylation of a protein of interest produced by the mammalian producer cell. Preferably, the extent of galactosylation, in particular the extent of terminal N-linked galactosylation, of the protein of interest is reduced by at least 5%, preferably at least 6%, preferably at least 7%, preferably at least 8%, preferably at least 9%, preferably at least 10%, preferably at least 15%, preferably at least 20%, preferably at least 25%, preferably at least 30%, compared to a protein of interest produced in a mammalian producer cell without a modulated level of at least one miRNA selected from the group consisting of miRNAs being capable of modifying the extent of galactosylation of a protein of interest produced by the mammalian producer cell.

In another preferred embodiment of the present invention, the protein of interest obtained in step c) has an increased extent of galactosylation, in particular extent of terminal N-linked galactosylation, compared to a protein of interest produced in a mammalian producer cell without a modulated level of at least one miRNA selected from the group consisting of miRNAs being capable of modifying the extent of galactosylation of a protein of interest produced by the mammalian producer cell. Preferably, the extent of galactosylation, in particular the extent of terminal N-linked galactosylation, of the protein of interest is increased by at least 5%, preferably at least 6%, preferably at least 7%, preferably at least 8%, preferably at least 9%, preferably at least 10%, preferably at least 15%, preferably at least 20%, preferably at least 25%, preferably at least 30%, compared to a protein of interest produced in a mammalian producer cell without a modulated level of at least one miRNA selected from the group consisting of miRNAs being capable of modifying the extent of galactosylation of a protein of interest produced by the mammalian producer cell.

Particularly preferred, the modulation in step b) is an increase in the level of at least one miRNA capable of modifying the extent of galactosylation of a protein of interest produced by the mammalian producer cell and the extent of galactosylation, in particular the extent of terminal N-linked galactosylation, of the protein of interest obtained in step c) is reduced, preferably reduced by at least 5%, preferably at least 6%, preferably at least 7%, preferably at least 8%, preferably at least 9%, preferably at least 10%, preferably at least 15%, preferably at least 20%, preferably at least 25%, preferably at least 30%, compared to a protein of interest produced in a mammalian producer cell without a modulated level of at least one miRNA selected from the group consisting of miRNAs being capable of modifying the extent of galactosylation of a protein of interest produced by the mammalian producer cell.

In another preferred embodiment of the present invention, the modulation in step b) is an increase in the level of at least one miRNA capable of modifying the extent of galactosylation of a protein of interest produced by the mammalian producer cell and the extent of galactosylation, in particular the extent of terminal N-linked galactosylation, of the protein of interest obtained in step c) is increased, preferably increased by at least 5%, preferably at least 6%, preferably at least 7%, preferably at least 8%, preferably at least 9%, preferably at least 10%, preferably at least 15%, preferably at least 20%, preferably at least 25%, preferably at least 30%, compared to a protein of interest produced in a mammalian producer cell without a modulated level of at least one miRNA selected from the group consisting of miRNAs being capable of modifying the extent of galactosylation of a protein of interest produced by the mammalian producer cell.

In a preferred embodiment, the modulation in step b) is a reduction in the level of at least one miRNA capable of modifying the extent of galactosylation of a protein of interest produced by the mammalian producer cell and the extent of galactosylation, in particular the extent of terminal N-linked galactosylation, of the protein of interest obtained in step c) is reduced, preferably reduced by at least 5%, preferably at least 6%, preferably at least 7%, preferably at least 8%, preferably at least 9%, preferably at least 10%, preferably at least 15%, preferably at least 20%, preferably at least 25%, preferably at least 30%, compared to a protein of interest produced in a mammalian producer cell without a modulated level of at least one miRNA selected from the group consisting of miRNAs being capable of modifying the extent of galactosylation of a protein of interest produced by the mammalian producer cell.

In a particularly preferred embodiment of the present invention, the modulation in step b) is a reduction in the level of at least one miRNA capable of modifying the extent of galactosylation of a protein of interest produced by the mammalian producer cell and the extent of galactosylation, in particular the extent of terminal N-linked galactosylation, of the protein of interest obtained in step c) is increased, preferably increased by at least 5%, preferably at least 6%, preferably at least 7%, preferably at least 8%, preferably at least 9%, preferably at least 10%, preferably at least 15%, preferably at least 20%, preferably at least 25%, preferably at least 30%, compared to a protein of interest produced in a mammalian producer cell without a modulated level of at least one miRNA selected from the group consisting of miRNAs being capable of modifying the extent of galactosylation of a protein of interest produced by the mammalian producer cell.

In a preferred embodiment of the present invention, the protein of interest, in particular the protein of interest having a desired extent of galactosylation obtained in step c), is an antibody or a fragment thereof.

In a further preferred embodiment of the present invention, the extent of galactosylation is the extent of terminal galactosylation, in particular the extent of terminal N-linked galactosylation or the extent of terminal O-linked galactosylation, preferably the extent of terminal N-linked galactosylation.

The present invention further pertains to a method for the preparation of a protein production engineered mammalian producer cell capable of expressing a protein of interest, comprising the steps:
x) providing a mammalian producer cell,
y) modulating in the mammalian producer cell provided in step x) the level of at least one miRNA selected from the group consisting of miRNAs being capable of modifying the extent of galactosylation of a protein produced by the mammalian producer cell so as to obtain a protein production engineered mammalian producer cell,
z) recovering the protein production engineered mammalian producer cell produced in step y).

By the method for the preparation of a protein production engineered mammalian producer cell capable of expressing a protein of interest a mammalian producer cell is obtained which is engineered to produce a protein of interest having a modified extent of galactosylation, in particular an increased or reduced extent of galactosylation. The protein production engineered mammalian producer cell prepared by the method according to the present invention is characterized by having a modulated level of at least one miRNA selected from the group consisting of miRNAs being capable of modifying the extent of galactosylation of a protein produced by the mammalian producer cell. Due to the modulated level of at least one miRNA, the protein production engineered mammalian producer cell advantageously provides a protein of interest with an extent of galactosylation differing from the extent of galactosylation which is obtained in absence of a modulated level of the at least of miRNA.

In a preferred embodiment of the present invention, the modulation of the level of at least one miRNA selected from the group consisting of miRNAs being capable of modifying the extent of galactosylation of a protein produced by the mammalian producer cell in step y) is an increase in the level of the at least one miRNA, in particular an increase of at least two fold, preferably at least three fold, preferably at least four fold, preferably at least five fold, preferably at least six fold, preferably at least seven fold, preferably at least eight fold, preferably at least nine fold, preferably at least 10-fold, preferably at least 15-fold, preferably at least 20-fold, preferably at least 25-fold, in comparison to the level of the at least one miRNA in a mammalian producer cell without a modulated level of the at least one miRNA.

In another preferred embodiment of the present invention, the modulation of the level of at least one miRNA selected from the group consisting of miRNAs being capable of modifying the extent of galactosylation of a protein produced by the mammalian producer cell in step y) is a reduction in the level of the at least one miRNA, in particular a reduction of at least two fold, preferably at least three fold, preferably at least four fold, preferably at least five fold, preferably at least six fold, preferably at least seven fold, preferably at least eight fold, preferably at least nine fold, preferably at least 10-fold, preferably at least 15-fold, preferably at least 20-fold, preferably at least 25-fold, in comparison to the level of the at least one miRNA in a mammalian producer cell without a modulated level of the at least one miRNA.

In a particularly preferred embodiment of the present invention, the level of at least two miRNAs, preferably at least three miRNAs, preferably at least four miRNAs, capable of modifying the extent of galactosylation of a protein produced by the mammalian producer cell is modulated, preferably reduced, preferably increased, in step b) or y) of the methods according to the present invention.

In another preferred embodiment, the level of at least one miRNA, preferably at least two miRNAs, preferably at least three miRNAs, capable of modifying the extent of galactosylation of a protein produced by the mammalian producer cell is increased and the level of at least one miRNA, preferably at least two miRNAs, preferably at least three miRNAs, capable of modifying the extent of galactosylation of a protein produced by the mammalian producer cell is reduced in step b) or y) of the methods according to the present invention.

In a particularly preferred embodiment of the present invention, the miRNA is a mature miRNA, a precursor miRNA or a primary miRNA. Preferably, the miRNA is a synthetic molecule, in particular a synthetic precursor miRNA.

Preferably, the at least one miRNA capable of modifying the extent of galactosylation of a protein produced by the mammalian producer cell is a miRNA selected from the group consisting of SEQ ID No. 1, SEQ ID No. 2, SEQ ID No. 3, SEQ ID No. 4 and SEQ ID No. 5.

In a particularly preferred embodiment of the present invention, the miRNA capable of modifying the extent of galactosylation of a protein produced by the mammalian producer cell comprises the nucleotide sequence of SEQ ID No. 1, preferably consist thereof.

In a further preferred embodiment of the present invention, the miRNA capable of modifying the extent of galactosylation of a protein produced by the mammalian producer cell comprises, in particular consists of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 1.

Preferably, the miRNA capable of modifying the extent of galactosylation of a protein produced by the mammalian producer cell comprises the nucleotide sequence of SEQ ID No. 2, preferably consist thereof.

In another preferred embodiment of the present invention, the miRNA capable of modifying the extent of galactosylation of a protein produced by the mammalian producer cell comprises, in particular consists of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 2.

In a further preferred embodiment of the present invention, the miRNA capable of modifying the extent of galactosylation of a protein produced by the mammalian producer cell comprises the nucleotide sequence of SEQ ID No. 3, preferably consist thereof. Preferably, the miRNA capable of modifying the extent of galactosylation of a protein produced by the mammalian producer cell is miR-30c or a homologue thereof.

In a preferred embodiment of the present invention, the miRNA capable of modifying the extent of galactosylation of a protein produced by the mammalian producer cell comprises, in particular consists of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 3.

Preferably, the miRNA capable of modifying the extent of galactosylation of a protein produced by the mammalian producer cell comprises the nucleotide sequence of SEQ ID No. 4, preferably consist thereof. Preferably, the miRNA capable of modifying the extent of galactosylation of a protein produced by the mammalian producer cell is miR-30e or a homologue thereof.

In a particularly preferred embodiment of the present invention, the miRNA capable of modifying the extent of galactosylation of a protein produced by the mammalian producer cell comprises, in particular consists of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 4.

In another preferred embodiment of the present invention, the miRNA capable of modifying the extent of galactosylation of a protein produced by the mammalian producer cell comprises the nucleotide sequence of SEQ ID No. 5, preferably consist thereof. Preferably, the miRNA capable of modifying the extent of galactosylation of a protein produced by the mammalian producer cell is miR-16 or a homologue thereof.

Preferably, the miRNA capable of modifying the extent of galactosylation of a protein produced by the mammalian producer cell comprises, in particular consists of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 5.

Preferably, the level of at least one miRNA is modulated by transfecting the mammalian producer cell with at least one transgenic nucleotide sequence encoding at least one miRNA capable of modifying the extent of glycosylation of a protein produced by the mammalian producer cell.

In a preferred embodiment of the present invention, the level of at least one miRNA is modulated by transfecting the mammalian producer cell in step b) or y) with at least one miRNA being capable of modifying the extent of glycosylation of a protein produced by the mammalian producer cell.

In another preferred embodiment of the present invention, the level of at least one miRNA is modulated by transfecting the mammalian producer cell in step b) or y) with at least one vector containing a nucleotide sequence encoding at least one miRNA under control of at least one regulatory element allowing the over-expression of the at least one miRNA, wherein the at least one miRNA is capable of modifying the extent of galactosylation of a protein produced by the mammalian producer cell.

In a particular embodiment of the present invention, the at least one vector containing a nucleotide sequence encoding at least one miRNA under control of at least one regulatory element allowing the over-expression of the at least miRNA is transiently or stably transfected, preferably transiently transfected, preferably stably transfected.

Transfection of the mammalian producer cell with the at least one miRNA being capable of modifying the extent of glycosylation of a protein produced by the mammalian producer cell, with at least one transgenic nucleotide sequence encoding at least one miRNA, in particular at least one vector containing a nucleotide sequence encoding at least one miRNA under control of at least one regulatory element allowing the over-expression of the at least one miRNA, and/or with a nucleotide sequence encoding the protein of interest can be mediated with any suitable transfection method known in the art, such as a liposome-based method, transfection using electroporation or by calcium phosphate-mediated transfection.

Preferably, the regulatory element allowing the over-expression of the at least one miRNA is a promotor. In a preferred embodiment of the present invention, the regulatory element allowing the over-expression of the at least one miRNA is an inducible promotor. In a particularly preferred embodiment, the regulatory element allowing the over-expression of the at least one miRNA is a temperature inducible promotor. In another preferred embodiment of the present invention, the regulatory element allowing the over-expression of the at least one miRNA is a constitutive promotor.

In a preferred embodiment of the present invention, the mammalian producer cell is transfected with a nucleotide sequence encoding the protein of interest prior to step a) or x) or during step b) or y). Preferably, the mammalian producer cell is transfected with a nucleotide sequence encoding the protein of interest prior to step a) or x). In another preferred embodiment, the mammalian producer cell is transfected with a nucleotide sequence encoding the protein of interest during step b) or y). Particularly preferred, the mammalian producer cell, in particular the protein production engineered mammalian producer cell, is transfected with a nucleotide sequence encoding the protein of interest after step b) or y).

In a preferred embodiment of the present invention, the mammalian producer cell is a CHO-cell, a BHK cell or a HEK293-cell. Particularly preferred, the mammalian producer cell is a CHO-cell. Preferably, the CHO-cell is selected from the group consisting of a CHO-DUXB 11 cell, CHO-DG44 cell and CHO-K1 cell.

In a further preferred embodiment of the present invention, the protein production engineered mammalian producer cell is a cell exhibiting an increased specific production rate or an increased time integral of viable cell concentration or both. Preferably, the protein production engineered mammalian producer cell is a cell exhibiting an increased specific production rate. Preferably, the protein production engineered mammalian producer cell is a cell exhibiting an increased time integral of viable cell concentration. Particularly preferred, the protein production engineered mammalian producer cell is a cell exhibiting an increased specific production rate and an increased time integral of viable cell concentration.

The present invention further pertains to a protein obtainable, preferably obtained, by the method for the production of a protein of interest according to the present invention.

According to the present invention, the protein obtainable, preferably obtained, by the method for the production of a protein of interest according to the present invention has an extent of galactosylation, preferably terminal N-linked galactosylation, which differs from the extent of galactosylation, preferably terminal N-linked galactosylation, of a protein of interest obtained without modulation of the level of at least one miRNA selected from the group consisting of miRNAs being capable of modifying the extent of galactosylation in the mammalian producer cell.

The present invention also pertains to a protein production engineered mammalian producer cell obtainable, preferably obtained, by the method for the preparation of a protein production engineered mammalian producer cell capable of expressing a protein of interest according to present invention.

The invention is further directed to a protein production engineered mammalian producer cell comprising at least one transgenic nucleotide sequence encoding at least one miRNA, which transgenic nucleotide sequence is transiently or stably incorporated into the genome of that cell under control of at least one regulatory element allowing the over-expression of said at least one miRNA, wherein the at least one miRNA is capable of modifying the extent of galactosylation of a protein produced by the mammalian producer cell.

The protein production engineered mammalian producer cells according to the present invention advantageously allows the production of a protein of interest having an extent of galactosylation, preferably terminal N-linked galactosylation, which differs from the extent of galactosylation, preferably terminal N-linked galactosylation, of the protein of interest produced by a protein production engineered mammalian producer cell without modulation of the level of at least one miRNA selected from the group consisting of miRNAs being capable of modifying the extent of galactosylation in the mammalian producer cell.

The present invention also encompasses a kit for the production of a protein of interest in a mammalian producer cell, comprising at least one mammalian producer cell, at least one vector containing a nucleotide sequence encoding at least one miRNA under control of at least one regulatory element allowing the over-expression of the at least one miRNA, wherein the at least one miRNA is capable of modifying the extent of galactosylation of a protein produced by the mammalian producer cell and means for transfecting the at least one cell with the at least one vector.

The present invention is also directed to a kit for modulating the extent of galactosylation, in particular terminal galactosylation, preferably terminal N-linked galactosylation, of a protein produced by a mammalian producer cell, comprising at least one buffer solution, at least one RNAse inhibitor, at least one miRNA capable of modifying the extent of galactosylation of a protein produced by a mammalian producer cell, and means for transfecting a mammalian producer cell with the at least one miRNA.

In a preferred embodiment, at least one miRNA capable of modifying the extent of galactosylation of a protein produced by a mammalian producer cell is an miRNA molecule comprising, in particular consisting of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 1, SEQ ID No. 2, SEQ ID No. 3, SEQ ID No. 4 or SEQ ID No. 5.

In a particularly preferred embodiment, the at least one miRNA capable of modifying the extent of galactosylation of a protein produced by a mammalian producer cell is an miRNA molecule selected from the group consisting of SEQ ID No. 1, SEQ ID No. 2, SEQ ID No. 3, SEQ ID No. 4 and SEQ ID No. 5.

In a preferred embodiment, the at least one miRNA capable of modifying the extent of galactosylation of a protein produced by a mammalian producer cell is lyophilized. Preferably, the at least one miRNA capable of modifying the extent of galactosylation of a protein produced by a mammalian producer cell is solubilized, in particular solubilized in a buffer solution.

In another aspect, the present invention is also directed to a kit for modulating the extent of galactosylation, in particular terminal galactosylation, preferably terminal N-linked galactosylation, of a protein produced by a mammalian producer cell, comprising at least one vector containing a nucleotide sequence encoding at least one miRNA capable of modifying the extent of galactosylation of a protein produced by the mammalian producer cell under control of at least one regulatory element allowing the over-expression of the at least one miRNA and means for transfecting a mammalian producer cell with the at least one vector.

In a preferred embodiment, at least one miRNA capable of modifying the extent of galactosylation of a protein produced by a mammalian producer cell is an miRNA molecule comprising, in particular consisting of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 1, SEQ ID No. 2, SEQ ID No. 3, SEQ ID No. 4 or SEQ ID No. 5.

In a particularly preferred embodiment, the at least one miRNA capable of modifying the extent of galactosylation of a protein produced by a mammalian producer cell is an miRNA molecule selected from the group consisting of SEQ ID No. 1, SEQ ID No. 2, SEQ ID No. 3, SEQ ID No. 4 and SEQ ID No. 5.

The invention further pertains to an miRNA molecule comprising, in particular consisting of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 1 or SEQ ID No. 2, preferably an miRNA molecule comprising the sequence according to SEQ ID No. 1 or SEQ ID No. 2, in particular consisting of the sequence according to SEQ ID No. 1 or SEQ ID No. 2.

The invention is also directed to the use of an miRNA molecule for modulating the extent of galactosylation, in particular terminal galactosylation, preferably terminal N-linked galactosylation, of a protein, in particular to the use of an miRNA molecule comprising, in particular consisting of, a nucleotide sequence having at least 80%, preferably at least 85%, preferably at least 90%, preferably at least 95%, preferably at least 96%, preferably at least 97%, preferably at least 98%, preferably at least 99%, sequence identity with the nucleotide sequence of SEQ ID No. 1, SEQ ID No. 2, SEQ ID No. 3, SEQ ID No. 4 or SEQ ID No. 5, preferably an miRNA molecule selected from the group consisting of SEQ ID No. 1, SEQ ID No. 2, SEQ ID No. 3, SEQ ID No. 4 and SEQ ID No. 5, for modulating the extent of galactosylation, in particular terminal galactosylation, preferably terminal N-linked galactosylation, of a protein.

In a particularly preferred embodiment of the present invention, the at least one miRNA is lyophilised. In a further preferred embodiment, the at least one miRNA is solubilised, in particular provided in a buffered solution.

The expression "extent of galactosylation" as used in the context of the present invention means the quantitative degree of galactosylation. Accordingly, an "increased extent of galactosylation" of the protein of interest can mean that the protein of interest is galactosylated at all upon modulation of the level of at least one miRNA selected from the group consisting of miRNAs being capable of modifying the extent of galactosylation of a protein produced by the mammalian producer cell. The expression can also mean that the glycan structure of the protein of interest comprises more galactose units in the glycan structure upon modulation of the level of at least one miRNA selected from the group consisting of miRNAs being capable of modifying the extent of galactosylation of a protein produced by the mammalian producer cell than without such modulation. Accordingly, the expression "modifying the extent of galactosylation" is used in the context of the present invention to designate an alternation in the quantitative degree of galactosylation of a protein of interest with respect to a situation in which no modulation of the level of the at least one miRNA in a mammalian producer cell expressing the protein of interest occurred.

In the context of the present invention the expressions "modulating the level of at least one miRNA" and corresponding grammatical forms thereof describe a change of the intracellular concentration of at least one miRNA, in particular an increase or reduction of the intracellular concentration of at least one miRNA, which is artificially caused, in particular induced. The modulation can for example be a targeted up-regulation of at least one miRNA, in particular an over-expression of the at least one miRNA, or a targeted down-regulation of at least one miRNA in the mammalian producer cell. The target up- or down-regulation of at least one miRNA can be effected by any suitable means in the art, such as by inducible or constitutive over-expression of at least one miRNA or knockdown or knockout of at least one miRNA.

According to the present invention, the expression "conditions suitable for the production of a protein of interest" means conditions, such as temperature, pressure, time, light, composition of the culture medium as well as presence or absence of inducers/repressors, which maintain, induce or enhance the expression of a target protein. The specific conditions required to achieve an efficient expression of the respective protein are known to the skilled person and *inter alia* depend on the mammalian producer cell used as the expression system and the suitable expression cassette, in particular its promotor.

In the context of the present invention the term "over-expression" refers to the artificial expression of a nucleic acid molecule, in particular an miRNA and/or a gene encoding a protein of interest, in increased quantity. This includes the inducible over-expression of a nucleic acid molecule, in particular an miRNA and/or a gene encoding a protein of interest, which can be regulated using an inducible promoter and the constant over-expression of a nucleic acid molecule, in particular an miRNA and/or a gene encoding a protein of interest, using a constitutive promoter.

The term "down-regulation" designates the artificial reduction of the level of a nucleic acid molecule, in particular an miRNA and/or a gene encoding a protein of interest.

In the context of the present invention, the term "a" is meant to include the meaning of "one" or "one or more".

In the context of the present invention, the term "comprising" preferably has the meaning of "containing" or "including", meaning including the specifically identified elements without excluding the presence of further elements. However, in a preferred embodiment, the term "comprising" is also understood to have the meaning of "consisting essentially of' and in a further preferred embodiment the meaning of "consisting of'.

The terms "and/or" is used herein to express the disclosure of any combination of individual elements connected within a listing. Solely for illustrative purposes, the expression "A, B, and/or C" can mean A individually, B individually, C individually, (A and B), (B and C), (A and C), and (A, B and C).

Further preferred embodiments of the invention are subject of the subclaims.

The invention is further described by way of the following figures and example.
**Figure 1** shows a comparison of the galactosylation of different therapeutic antibodies (originators) and their biosimilars.
**Figure 2** shows different glycosylation patterns of terminal N-linked glycans of monoclonal antibodies.
**Figure 3** shows the effect of miRNA mimics (SEQ ID No. 1 to 5 and their respective antisense strand) on the productivity of CHO DG44 cells expressing Adalimumab and on the extent of galactosylation after transient transfection with the miRNA mimics. Anti-IgG miR was transfected as technical control. n = 3 for negative control, anti-IgG miR, n = 2 for all other miRNAs, one-way ANOVA was performed for statistical analysis with ^{∗} p < 0.05, ^{∗∗} p < 0.01, ^{∗∗∗} p < 0.001, ^{∗∗∗∗} p < 0.0001, error indicates standard deviation.

### Example:

### Reduction of galactosylation upon transient miRNA mimic transfection

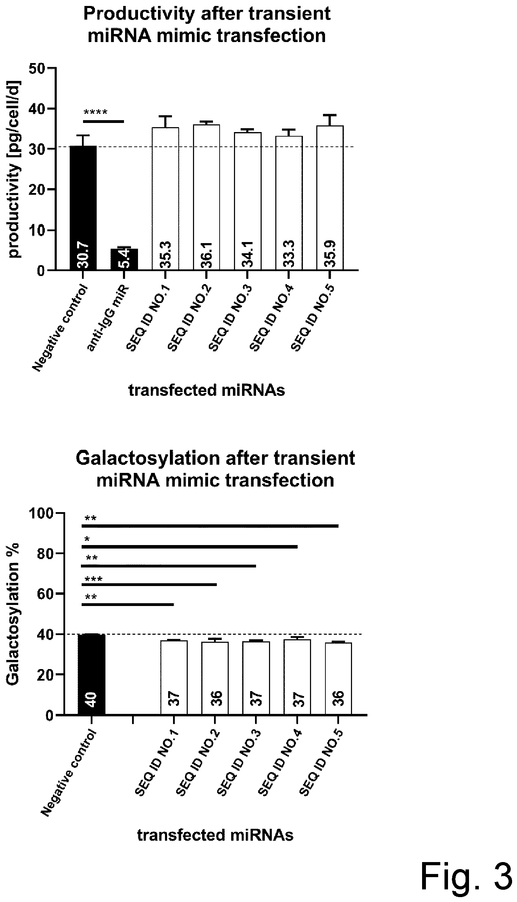

In order to evaluate miRNA-mediated effects on productivity and N-glycosylation, mimics of five different miRNAs (SEQ ID No. 1 to 5 including their respective antisense strand) were transiently transfected at 100 nM concentration to CHO DG44 cells producing Adalimumab. It could be observed that transfection with the five miRNA mimics did not significantly reduce productivity of CHO DG44 cells, but significantly reduced the extent of galactosylation (see Fig. 3).

Transfection of CHO DG44 cells with miRNA mimics of either SEQ ID No. 1, SEQ ID No. 3 or SEQ ID No. 4 reduced the portion of recombinantly produced Adalimumab with terminal galactose glycans from 40% (negative control) to 37%. Transfection of CHO DG44 cells with miRNA mimics of either SEQ ID No. 2 or SEQ ID No. 5 resulted in a reduction of the portion of recombinantly produced Adalimumab with terminal galactose glycans from 40% (negative control) to 36%.

**Table 1: Effect of miRNA mimic transfection on the extent of galactosylation**

| **SEQ ID No.** | *miRNA sequence* | *Galactosylation of control (%)* | *Galactosylation after miRNA mimic transfection (%)* | *Change in Galactosylation (%)* |
|---|---|---|---|---|
| **1** | agggcucgggcucuacuccagg | 40 | 37 | -7.5 |
| **2** | gguacaagccgagcccgacug | 40 | 36 | -10 |
| **3** | uguaaacauccuacacucucagc | 40 | 37 | -7.5 |
| **4** | uguaaacauccuugacuggaagc | 40 | 37 | -7.5 |
| **5** | uagcagcacguaaauauuggc | 40 | 36 | -10 |

## Claims

1. A method for the production of a protein of interest comprising the steps:
a) providing a mammalian producer cell,
b) modulating in the mammalian producer cell provided in step a) the level of at least one miRNA selected from the group consisting of miRNAs being capable of modifying the extent of galactosylation of a protein of interest produced by the mammalian producer cell,
c) cultivating the mammalian producer cell with the modulated level of the at least one miRNA of step b) under conditions suitable for the production of the protein of interest so as to obtain the protein of interest having a desired extent of galactosylation.

2. The method according to claim 1, further comprising step d) of recovering the protein of interest produce in step c).

3. The method according to claim 1, wherein in step b) the level of at least one miRNA selected from the group consisting of miRNAs being capable of modifying the extent of galactosylation of a protein is increased.

4. The method according to anyone of claims 1 to 3, wherein the protein of interest obtained in step c) has a reduced extent of galactosylation compared to a protein of interest produced in a mammalian producer cell without a modulated level of at least one miRNA selected from the group consisting of miRNAs being capable of modifying the extent of galactosylation of a protein produced by the mammalian producer cell.

5. A method for the preparation of a protein production engineered mammalian producer cell capable of expressing a protein of interest, comprising the process steps
x) providing a mammalian producer cell,
y) modulating in the mammalian producer cell provided in step x) the level of at least one miRNA selected from the group consisting of miRNAs being capable of modifying the extent of galactosylation of a protein produced by the mammalian producer cell so as to obtain a protein production engineered mammalian producer cell,
z) recovering the protein production engineered mammalian producer cell produced in step y).

6. The method according to any one of the preceding claims, wherein the at least one miRNA being capable of modifying the extent of galactosylation of a protein produced by the mammalian producer cell is an miRNA selected from the group consisting of SEQ ID No. 1, SEQ ID No. 2, SEQ ID No. 3, SEQ ID No. 4 and SEQ ID No. 5.

7. The method according to any one of the preceding claims, wherein the level of the at least one miRNA is modulated by transfecting the mammalian producer cell in step b) or y) with at least one miRNA being capable of modifying the extent of galactosylation of a protein produced by the mammalian producer cell.

8. The method according to any one of claims 1 to 6, wherein the level of the at least one miRNA is modulated by transfecting the mammalian producer cell in step b) or y) with at least one vector containing a nucleotide sequence encoding at least one miRNA under control of at least one regulatory element allowing the over-expression of the at least one miRNA, wherein the at least one miRNA is capable of modifying the extent of galactosylation of a protein produced by the mammalian producer cell.

9. The method according to claim 7 or 8, wherein at least two of the miRNAs or at least two of the nucleotide sequences encoding at least two of the miRNAs are transfected.

10. The method according to any one of the preceding claims, wherein the miRNA is a mature miRNA, a precursor miRNA or a primary miRNA.

11. The method according to any one of claims 8 to 10, wherein the at least one vector encoding the at least one miRNA in step b) or y) is transiently or stably transfected.

12. The method according to any one of the preceding claims, wherein the mammalian producer cell is transfected with a nucleotide sequence encoding the protein of interest prior to step a) or x) or during step b) or y).

13. The method according to any one of claims 1 to 11, wherein the protein production engineered mammalian producer cell is transfected with a nucleotide sequence encoding the protein of interest after step b) or y).

14. The method according to any one of the preceding claims, wherein the mammalian producer cell is a CHO-cell.

15. The method according to any one of claims 8 to 14, wherein the regulatory element allowing the over-expression of the at least one miRNA is a promoter.

16. The method according to any one of the preceding claims, wherein the protein production engineered mammalian producer cell is a cell exhibiting an increased specific production rate or an increased time integral of viable cell concentration or both.

17. Protein, obtainable in a method according to anyone of the methods of claims 1 to 16.

18. A protein production engineered mammalian producer cell produced by a method according to anyone claims 5 to 16.

19. A protein production engineered mammalian producer cell comprising at least one transgenic nucleotide sequence encoding at least one miRNA, which transgenic nucleotide sequence is transiently or stably incorporated into the genome of that cell under control of at least one regulatory element allowing the over-expression of said at least one miRNA, wherein the at least one miRNA is capable of modifying the extent of galactosylation of a protein produced by the mammalian producer cell.

20. A kit for the production of a protein of interest in a mammalian producer cell, comprising at least one mammalian producer cell, at least one vector containing a nucleotide sequence encoding at least one miRNA under control of at least one regulatory element allowing the over-expression of the at least one miRNA, wherein the at least one miRNA is capable of modifying the extent of galactosylation of a protein produced by the mammalian producer cell and means for transfecting the at least one cell with the at least one vector.

21. A kit for modulating the extent of galactosylation, in particular terminal galactosylation, of a protein produced by a mammalian producer cell, comprising at least one buffer solution, at least one RNAse inhibitor, at least one miRNA capable of modifying the extent of galactosylation of a protein produced by a mammalian producer cell, and means for transfecting a mammalian producer cell with the at least one miRNA.

22. An miRNA molecule having the sequence according to SEQ ID No. 1 or SEQ ID No. 2.

23. Use of an miRNA molecule for modulating the extent of galactosylation of a protein.
